# EUROPEAN PATENT SPECIFICATION

(11) **EP 0 205 711 B1**
(45) Date of publication and mention of the grant of the patent: **07.01.1993**
(21) Application number: 85309347.4
(22) Date of filing: 20.12.1985
(51) Int. Cl.: C01B 33/20, B01J 21/06

(54) **Layered oxides containing interlayer polymeric oxides and their synthesis**
Zwischenschichten von polymeren Oxiden enthaltende Schichtoxide und ihre Synthese
Oxydes en couches contenant des couches intermédiaires d'oxydes polymères et procédé pour les préparer

(30) Priority: 28.12.1984 US 687414
(43) Date of publication of application: 30.12.1986
(73) Proprietor: MOBIL OIL CORPORATION, New York New York 10017 (US)
(72) Inventor: Chu, Pochen, West Deptford New Jersey 08066 (US); Landis, Michael Eugene, Woodbury New Jersey 08096 (US); Kirker, Garry Wayne, Sewell New Jersey 08080 (US)
(74) Representative: Cooper, John Anthony

(56) References cited:
- EP-A- 0 150 898
- US-A- 4 176 090
- US-A- 4 367 163
- US-A- 4 510 257
- US-A- 4 515 901

## Description

The present invention relates to layered oxides containing interlayer polymeric oxides and to their synthesis.

Many layered materials are known which have three-dimensional structures which exhibit their strongest chemical bonding in only two dimensions. In such materials, the stronger chemical bonds are formed in two-dimensional planes and a three-dimensional solid is formed by stacking such planes on top of each other. However, the interactions between the planes are weaker than the chemical bonds holding an individual plane together. The weaker bonds generally arise from interlayer attractions such as Van der Waals forces, electrostatic interactions, and hydrogen bonding. In those situations where the layered structure has electrically neutral sheets interacting with each other solely through Van der Waals forces, a high degree of lubricity is manifested as the planes slide across each other without encountering the energy barriers that arise with strong interlayer bonding. Graphite is an example of such a material. The silicate layers of a number of clay materials are held together by electrostatic attraction provided by ions located between the layers. In addition, hydrogen bonding interactions can occur directly between complementary sites on adjacent layers, or can be provided by interlamellar bridging molecules.

Layered materials such as clays may be modified to increase their surface area. In particular, the distance between the layers can be increased substantially by absorption of various swelling agents such as water, ethylene glycol, amines and ketones, which enter the interlamellar space and push the layers apart. However, the interlamellar spaces of such layered materials tend to collapse when the molecules occupying the space are removed by, for example, exposing the clays to high temperatures. Accordingly, such layered materials having enhanced surface area are not suited for use in chemical processes involving even moderately severe conditions.

The extent of interlayer separation can be estimated by using standard techniques such as X-ray diffraction to determine the basal spacing, also known as "repeat distance" or "d-spacing". These values indicate the distance between, for example, the uppermost margin of one layer and the uppermost margin of its adjoining layer. If the layer thickness is known, the interlayer spacing can be determined by subtracting the layer thickness from the basal spacing.

Various approaches have been taken to provide layered materials of enhanced interlayer distance having thermal stability. Most techniques rely upon the introduction of an inorganic "pillaring" agent between the layers of a layered material. For example, U.S. Patent 4,216,188 discloses a clay which is cross-linked with metal hydroxide prepared from a highly dilute colloidal solution containing fully separated unit layers and a cross-linking agent comprising a colloidal metal hydroxide solution. However, this method requires a highly dilute forming solution of the clay (less than 1g per liter) in order to effect full layer separation prior to incorporation of the pillaring species, as well as positively charged species of cross linking agents. U.S. Patent 4,248,739 relates to stable pillared interlayered clay prepared from smectite clays reacted with cationic metal complexes of metals such as aluminum and zirconium. The resulting products exhibit high interlayer separation and thermal stability.

U.S. Patent 4,176,090 discloses a clay composition interlayered with polymeric cationic hydroxy metal complexes of metals such as aluminum, zirconium and titanium. Interlayer distances of up to 16A are claimed although only distances restricted to about 9A are exemplified for calcined samples. These distances are essentially unvariable and depend on the specific size of the hydroxy metal complex.

Silicon-containing materials are believed to be a highly desirable species of pillaring agents owing to their high thermal stability. U.S. Patent 4,367,163, for example, describes a clay pillared with silica prepared by impregnating a clay substrate with a silicon-containing reactant such as an ionic silicon complex, e.g., silicon acetylacetonate, or a neutral species such as SiCl₄. The clay may be swelled prior to or during silicon impregnation with a suitable polar solvent such as methylene chloride, acetone, benzaldehyde, or dimethylsulfoxide. This method, however, appears to provide only a monolayer of intercalated silica resulting in a product of small spacing between layers, about 2-3 A as determined by X-ray diffraction.

In a first aspect, the present invention resides in a layered product comprising a layered oxide of an element ranging in atomic number from 13 to 15, 21 to 33, 39 to 51, 57 to 83 and greater than 90, inclusive, and pillars containing a polymeric oxide of an element selected from Group IVB of the Periodic Table separating the oxide layers, said composition having a d-spacing of at least 20A.

In a second aspect, the invention resides in a layered product comprising a non-swellable layered oxide of an element ranging in atomic number from 13 to 15, 21 to 33, 39 to 51, 57 to 83 and greater than 90, inclusive, and pillars containing at least one polymeric oxide separating the oxide layers.

In a third aspect, the invention resides in a layered titanate composition having pillars containing polymeric silica between the titanate layers and having the characteristic X-ray diffraction pattern of Table 1 below.

**TABLE 1**

| COMPOSITE LIST OF PRINCIPAL X-RAY POWDER DIFFRACTION PEAKS FOR SILICOTITANATES | | |
|---|---|---|
| Line Number | 2 theta min - 2 theta max | 100 I/Iₒ (Relative Intensity) Range |
| 1 | 8.7 | VS to W |
| 2 | 11.1 - 14.3 | S to W |
| 3 | 11.8 - 15.2 | M to W |
| 4 | 24.5 - 25.0 | VS to W |
| 5 | 25.0 - 25.4 | M to W |
| 6 | 28.5 - 30.2 | VS to W |
| 7 | 29.8 - 30.6 | S to W |
| 8 | 33.0 - 33.5 | S to W |
| 9 | 43.2 - 43.5 | M to W |
| 10 | 44.2 - 44.7 | M to W |
| 11 | 48.5 - 48.9 | VS to M |
| 12 | 52.7 - 52.9 | W |
| 2 theta min - 2 theta max = Range of 2 theta-values observed for eight specific pillared silicotitanates | | |

These values were determined by standard techniques.

The radiation was the K-alpha doublet of copper, and a scintillation counter spectrometer was used. The peak heights, I, and the positions as a function of 2 times theta, where theta is the Bragg angle, were determined. From these, the relative intensities, I/Iₒ where Iₒ is one hundredth of the intensity of the strongest line or peak, and d is the interplanar spacing in Angstroms (A), corresponding to the recorded lines, were calculated. The relative intensity in the table above is expressed as follows:

| Relative Intensity | 100 I/Iₒ |
|---|---|
| VS (Very Strong) | 60-100 |
| S (Strong) | 40-60 |
| M (Medium) | 20-40 |
| W (Weak) | 0-20 |

Minor variations in the interplanar spacing and relative intensity may occur as a result of ion exchange, changes in the composition of the silicotitanate, or exposure to calcination conditions.

In a fourth aspect, the invention resides in a method for preparing a layered product having adjacent layers separated by pillars of a polymeric oxide of an element selected from Groups IB, IIB, IIIA, IIIB, IVA, IVB, VA, VB, VIA, VIIA, and VIIIA of the Periodic Table, which method comprises starting with a layered oxide material of an element ranging in atomic number from 13 to 15, 21 to 33, 39 to 51, 57 to 83 and greater than 90, said layered oxide material having anionic sites associated therewith; physically separating the layers of the oxide material by introducing an organic cationic species between the layers at said anionic sites; introducing between the separated layers of said layers oxide at least one neutral compound capable of conversion to polymeric oxide; and converting said compound to the said polymeric oxide to produce pillars of the polymeric oxide separating adjacent layers of the layered oxide material.

The method of present invention is particularly useful in that it permits the preparation of layered oxide materials of relatively high d-spacing, e.g., greater than about 10A, preferably greater than about 20A, up to or even exceeding 30A, preferably up to 25A. These materials can be exposed to severe conditions such as those encountered in calcining without significant decrease in interlayer distance. Furthermore, such layered oxides can be prepared without the severe dilution necessary to introduce the pillaring material as is often encountered in prior art techniques of interlayering. Finally, by varying the size of the organic cationic species separating the oxide layers, it is possible to form pillared products with widely varying interlayer spacing.

The method of the present invention utilizes a layered oxide starting material which has interlayer cations associated therewith. Such cations may include hydrogen ion, hydronium ion and alkali metal cations. The starting material is then treated with a "propping" agent comprising a source of an organic cation, such as an organoammonium cation, in order to effect an exchange of or addition to the interlayer cations resulting in the layers of the starting material being propped apart. The source of organic cation in those instances where the interlayer cations include hydrogen or hydronium ions may include a neutral compound such as an organic amine which is converted to a cationic analogue during the "propping" treatment. The foregoing treatment results in the formation of a layered metal oxide of enhanced interlayer separation depending upon the size of the organic cation introduced. In one embodiment, a series of organic cation exchanges is carried out. For example, an organic cation may be exchanged with an organic cation of greater size, thus increasing the interlayer separation in a step-wise fashion. Preferably, contact of the layered oxide with the propping agent is conducted in aqueous medium so that water is trapped within the interlayer spaces of the propped oxide.

After the ion exchange, the organic-"propped" species is treated with a compound capable of conversion, preferably by hydrolysis, to a polymeric oxide. The "propped" layered material containing the polymeric oxide precursor is then treated to produce polymeric oxide pillars separating the oxide layers. Where the treatment involves hydrolysis, this may for example be carried out using water already present in organic-"propped" layered oxide material.

It is preferred that the organic cation deposited between the layers be capable of being removed from the layered oxide material without substantial disturbance or removal of the polymeric oxide or oxide precursor. For example, organic cations such as n-octylammonium may be removed by calcination or chemical oxidation, preferably by calcination and preferably after the polymeric oxide precursor has been converted to the polymeric oxide.

The resulting oxide-pillared product exhibits high surface area, e.g., greater than 200, 400, or even 600 m²/g, and thermal stability making it useful as a catalyst or catalytic support for hydrocarbon conversion processes, for example cracking and hydrocracking.

The layered oxides used in the present invention are layered oxides of elements having an atomic number from 13 to 15, 21 to 33, 39 to 51, 57 to 83 or greater than 90, Preferably, the layered oxide is "non-swellable" which is intended to distinguish from conventional clays which contain octahedrally coordinated metal oxide sheets bonded to tetrahedrally coordinated silica sheets and which undergo substantial swelling, sometimes by an essentially unbounded amount, when contacted with water. As used herein in relation to a layered oxide material, the term "non-swellable" is defined as meaning a layered oxide material which, when contacted with at least 10 grams of water per gram of the layered oxide at 23^{o}C for 24 hours, exhibits an increase in d-spacing no greater than 5A as compared with the anyhydrous material. Included among these materials are H₂Ti₃O₇, Na₂Ti₃O₇ and KTiNbO₅ as well as certain layered silicates, for example, the metasilicates magadiite, natrosilite, kenyaite, makatite and kanemite. Other suitable starting materials include layered clays, such as bentonite, although these are swellable in water. Where the starting layered material is a layered silicate, it has been found to be preferable to treat the silicate with one or more polar solvents prior to or during exchange with the source of organic cation. The polar solvent used should exhibit electric dipole moments in the gas phase of at least 3.0 Debyes (D), preferably at least 3.5 D, most preferably at least about 3.8D. Examples of suitable solvents are water, dimethylsulfoxide (DMSO) and dimethylformamide (DMF). A table of selected organic compounds and their electric dipole moments can be found in CRC Handbook of Chemistry and Physics, 61st Edition, 1980-1981 at pages E-64 to E-66. It is believed that the treatment of the oxide starting material with one or more highly polar solvents facilitates the introduction of the source of organic cation between the layers of the starting material.

In one preferred embodiment, the starting material is a layered oxide of Group IV A metal such as titanium, zirconium and hafnium, with a layered titanate, e.g., a trititanate such as Na₂Ti₃O₇, being particularly preferred. Trititanates are commercially available materials whose structure consists of anionic sheets of titanium octahedra with interlayer alkali metal cations. A method for making such material may be found in U.S. Patent 2,496,993. It is known that the interlayer distance of Na₂Ti₃O₇ may be increased by replacing interlayer sodium ions with larger octylammonium ions. See, Weiss et al., Angew. Chem/72 Jahrg. 1960/Nr/2, pp 413-415. However, the organic-containing trititanate is highly susceptible to heat which can remove the organic material and cause collapse of the layered structure. The present invention serves to introduce a stable polymeric oxide, preferably silica, between adjoining layers resulting in a heat-stable material which substantially retains its interlayer distance upon calcination.

In another preferred embodiment, the oxide starting material is a layered silicate, such as magadiite, either in natural or synthetic form.

As previously stated, the starting layered oxide material is treated with an organic compound capable of forming cationic species such as organophosphonium or organoammonium ion, before adding the polymeric oxide source. Insertion of the organic cation between the adjoining layers serves to physically separate the layers in such a way as to make the layered oxide receptive to the interlayer addition of an electrically neutral, hydrolyzable, polymeric oxide precursor. In particular, alkylammonium cations have been found useful in the present invention. Thus C₃ and larger alkylammonium, e.g., n-octylammonium, cations are readily incorporated within the interlayer species of the layered oxides, serving to prop open the layers in such a way as to allow incorporation of the polymeric oxide precursor. The extent of the interlayer spacing can be controlled by the size of the organoammonium ion employed so that use of the n-propymonium cation will achieve a d-spacing of about 10.5A, whereas to achieve a d-spacing of 20A an n-octylammonium cation or a cation of equivalent length is required. Indeed, the size and shape of the organic cation can affect whether or not it can be incorporated within the layered oxide structure at all. For example, bulky cations such as tetrapropylammonium are generally undesirable for use in the present method, with ammonium cations derived from n-alkyl primary amines, more preferably primary monoamines, being preferred. The organic ammonium cations separating the oxide layers may be formed in situ by reaction of the neutral amine species with interlayer hydrogen or hydronium cations of the layer oxide starting material. Alternatively where the interlayer cations of the layered oxide starting material are alkali metal cations, the organic ammonium cation may be formed by initially combining an amine and an aqueous acid solution, such as hydrochloric acid, and then treating the layered oxide with the resulting aqueous organoammonium ion solution. In either case, the treatment is conducted in aqueous media so that water is then available to hydrolyze the electrically neutral, hydrolyzable polymeric oxide precursor subsequently introduced into the "propped" product.

The polymeric oxide pillars formed between the layers of the oxide starting material may include an oxide of zirconium or titanium or more preferably of an element selected from Croup IVB of the Periodic Table (Fischer Scientific Company Cat. No. 5-702-10), other than carbon, and most preferably include polymeric silica. In addition the polymeric oxide pillars may include an element which provides catalytically active acid sites in the pillars, preferably aluminum.

The polymeric oxide pillars are formed from a precursor material which is preferably introduced between the layers of the organic propped species as a cationic, or more preferably electrically neutral, hydrolyzable compound of the desired Group IVB elements. The precursor material is preferably an organometallic compound which is a liquid under ambient conditions. Suitable polymeric silica precursor materials include tetrapropylorthosilicate, tetramethylorthosilicate and, most preferably, tetraethylorthosilicate. Where the pillars are also required to include polymeric alumina, a hydrolyzable aluminum compound is contacted with the organic propped species before, after or simultaneously with the silicon compound. Preferably, the aluminum compound is aluminum isopropoxide.

After hydrolysis to produce the polymeric oxide pillars and calcination to remove the organic propping agent, the final pillared product may contain residual exchangeable cations. For example, sodium titanate pillared with polymeric silica may contain 2-3% of weight of residual sodium. Such residual cations can be ion exchanged by methods well known with other cationic species to provide or alter the catalytic activity of the pillared product. Suitable replacement cations include cesium, cobalt, nickel, copper, zinc, manganese, platinum, lanthanum, aluminum and mixtures thereof.

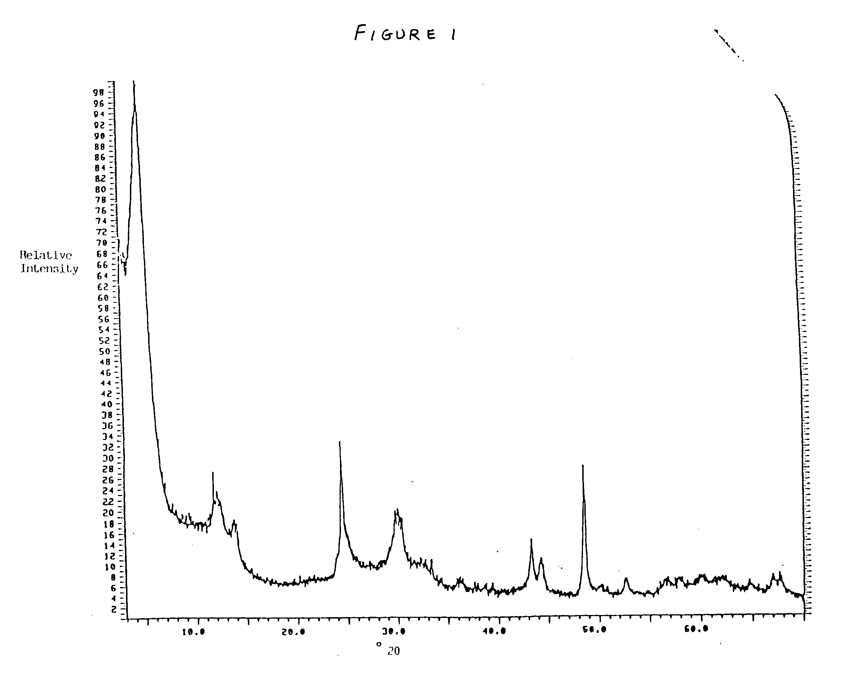

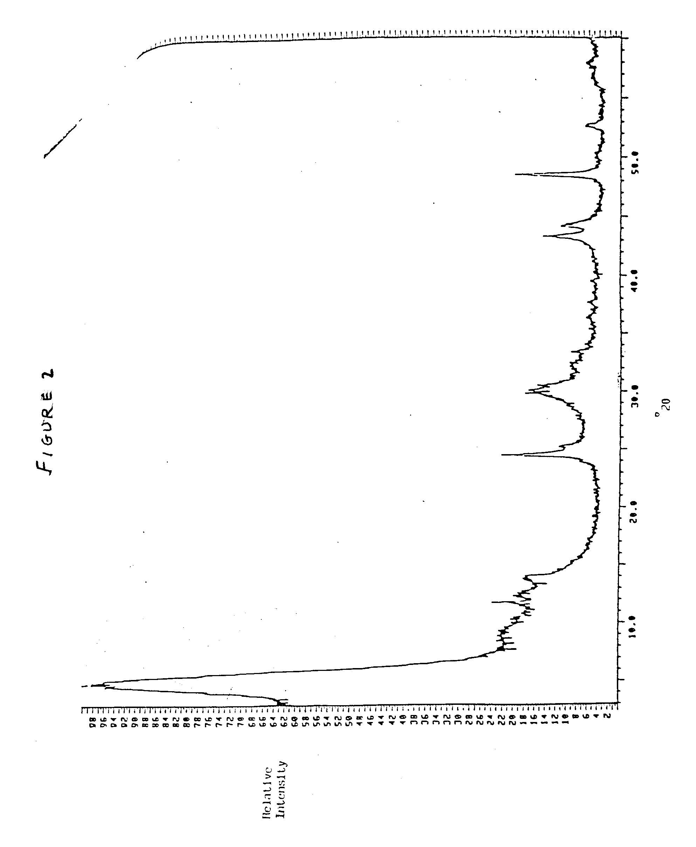

The present invention is illustrated further by the following examples. and the accompanying drawings which provide X-ray diffraction patterns of the silicotitanates of Example 1 (Figure 1) and Example 4 (Figure 2)

In these examples, adsorption data were determined as follows: A weighed sample was contacted with the desired pure adsorbate vapor at a pressure less than the vapor-liquid equilibrium pressure of the adsorbate at room temperature. Adsorption was complete when a constant pressure in the adsorption chamber was reached (overnight for water, 3 hours for hydrocarbons); e.g., 12 mm of mercury for water and 40 mm for n-hexane and cyclohexane. Samples were then removed and weighed. The increase in weight was calculated as the adsorption capacity of the samples. Nitrogen BET surface areas were reported in m²/g. X-ray diffraction data was obtained by standard techniques using K-alpha doublet of copper radiation.

When Alpha Value is examined, it is noted that the Alpha Value is an approximate indication of the catalytic cracking activity of the catalyst compared to a standard catalyst and it gives the relative rate constant (rate of normal hexane conversion per volume of catalyst per unit time). It is based on the activity of the highly active silica alumina cracking catalyst taken as an Alpha of 1 (Rate Constant = 0.16 sec⁻¹). The Alpha Test is described in U.S. Patent 3,354,078 and in The Journal of Catalysis, Vol. IV. pp. 522-529 (August 1965).

### EXAMPLE 1

A 20g sample of Na₂Ti₃O₇ (Alpha Products, Lot #101380) was calcined in air at 538°C (1000°F) for 1 hour. The product exhibited the following physical properties:
Surface Area: less than 5.0 m²/g
Sorption (g/100 g):

| | |
|---|---|
| H₂O | 0.3 |
| Cyclohexane | 0.4 |
| n-Hexane | 0.2 |

Thus, the starting titanate had a low surface area and negligible hydrocarbon sorptive capacity. The X-ray diffraction pattern of Na₂Ti₃O₇ indicated a layer thickness of about 7.4 angstoms.

300g of concentrated HCl (36.6%) were dissolved in 700g of water, and the resulting solution was placed in a 2 liter beaker, stirred with a magnetic stirrer, and cooled in ice. n-Octylamine (C₈H₁₇NH₂, 410g) was then added at a rate such that the solution temperature remained below 45°C. Sodium titanate (100g of Na₂Ti₃O₇) was added, and the mixture was transferred to a 2 liter polypropylene jar and heated at 100°C for 24 hours with occasional stirring. The product was filtered, washed with 3 liters of hot water and then 250 ml of absolute ethanol (room temperature), and finally 2 liters of hot water.

The dried product (110°C, 1 hour) had the following composition (mole ratios):

1.00 TiO₂:0.16C₈H₁₇NH₂:0.077Na₂O

The product (15.0g) was then stirred in 100g tetraethylorthosilicate in a 250 ml beaker covered with a watchglass for 3 days at room temperature. The product was filtered, dried at room temperature for 17 hours and then calcined in air for 3 hours in an oven pre-heated to 538°C (1000°F). The silicotitanate product had the x-ray diffraction pattern shown in Figure 1 and listed in Table 2 as well as the following composition (mole ratios):

1.00TiO₂:0.69SiO₂:0.068Na₂O

The product had a surface area of 289 m²/g and exhibited the following sorption characteristics (g/100g):

| | |
|---|---|
| H₂O | 9.8 |
| Cyclohexane | 8.8 |
| n-Hexane | 5.8 |

These data indicate that the production of polymeric silica pillars between the layers of the titanate can dramatically increase its surface area and sorptive behavior. The product had an alpha-value of 4.

**TABLE 2**

| TABULATION OF THE PRINCIPAL PEAKS IN THE X-RAY POWDER DIFFRACTION PATTERN OF THE PRODUCT OF EXAMPLE 1 | | | |
|---|---|---|---|
| Line Number | 2 theta | d(A) | 100 I/Iₘₐₓ (Relative Intensity) |
| 1 | 4.96 | 17.81 | 100 |
| 2 (Broad) | 12.19 | 7.26 | 15 |
| 3 (Broad | 13.88 | 6.38 | 12 |
| 4 | 24.52 | 3.63 | 78 |
| 5 (Shoulder) | 25.05 | 3.56 | 24 |
| 6 | 30.07 | 2.972 | 31 |
| 7 | 33.37 | 2.685 | 7 |
| 8 | 43.30 | 2.090 | 28 |
| 9 | 44.28 | 2.046 | 16 |
| 10 | 48.53 | 1.876 | 73 |
| 11 | 52.74 | 1.736 | 8 |

### EXAMPLE 2 (COMPARATIVE)

10g of the octylammonium propped titanate prepared in Example 1 was calcined in air at 538^{o}C (1000°F) for 3 hours without initial treatment with tetraethylorthosilicate . The product had a surface area of 10 m²/g and the following sorption characteristics (g/100g):

| | |
|---|---|
| H₂O | 0.6 |
| Cyclohexane | 0.7 |
| n-Hexane | 0.9 |

Thus, the tetraethylorthosilicate treatment was necessary to produce a material which retained its high surface area and sorptive capacity after calcination.

### EXAMPLE 3 (COMPARATIVE)

A mixture of 15.0g Na₂Ti₃O₇ in 100g tetraethylorthosilicate was stirred for 3 days at room temperature. The mixture was filtered and air dried for 24 hours at room temperature. The product had the following composition (mole ratios):

1.00TiO₂:0.012SiO₂:0.33Na₂O

The absence of significant levels of silica in this product indicate the necessity of pre-exchange with organic ammonium ions for silica-incorporation.

A portion of this product (7.0g) was calcined at 538^{o}(1000°F) in a pre-heated oven for 3 hours. The product had the following properties:
Surface Area less than 5.0 m²/g
Sorption (g/100g):

| | |
|---|---|
| H₂O | 0.2 |
| Cyclohexane | 0.3 |
| n-Hexane | 0.2 |

Thus, the absence of significant silica-incorporation produces a product with low surface area and negligible sorption capacity.

### EXAMPLE 4

320.8g of concentrated HCl (36.6%) were dissolved in 700g water and stirred in a 2 liter beaker. The mixture was cooled in ice and 427.1g n-octylamine were added at such a rate to keep the solution temperature below 50°C. After addition of the amine, 100g Na₂Ti₃O₇ were added, and the resulting mixture was transferred to a 2 liter polypropylene jar and heated with occasional stirring at 100°C for 24 hours. The product was filtered, and washed sequentially with 3 liters of hot water, 250 ml absolute ethanol (room temperature), and 2 liters of hot water. After drying for 1 hour 121°C (250°F), the product had the following composition (mole ratios):

1.00TiO₂:0.15C₈H₁₇NH₂:0.050Na₂O

The dried product (75g) was stirred with 500g tetraethylorthosilicate in a one liter beaker covered with a watchglass for 3 days at room temperature. The product was filtered, dried at room temperature for 24 hours and calcined in air in a pre-heated oven for 3 hours at 538°C (1000°F). The silico-titanate product had an x-ray diffraction pattern (Figure 2) similar to that observed for the product of Example 1 and had the following composition (mole ratios):

1.00TiO₂:0.23SiO₂:0.053Na₂O

The product had a surface area of 191 m²/g and exhibited the following sorption characteristics (g/100 g):

| | |
|---|---|
| H₂O | 9.5 |
| cyclohexane | 6.6 |
| n-hexane | 5.1 |

The product had an alpha-value of 3.

### EXAMPLE 5

Concentrated HCl (320.8g of 36.6% HCl) was dissolved in 700g water, and the resulting solution was stirred and cooled in an ice bath. n-Octylamine (427.1g) was added keeping the solution below 50°C. 100 grams of Na₂Ti₃O₇ were added, and the mixture was transferred to a 2 liter polypropylene jar and heated at 100°C with occasional stirring for 24 hours. Approximately one-half of the mixture was filtered, washed sequentially with 1.5 liters hot water, 125 ml absolute ethanol, and 1 liter hot water, and dried at room temperature for 24 hours.

15 grams of this product was stirred in 100g tetraethylorthosilicate at room temperature for 72 hours. The product was filtered, air-dried for 1 hour at room temperature, and calcined in air for 3 hours at 538°C (1000°F) in a pre-heated oven. The product had an x-ray diffraction pattern similar to those of Examples 1 and 4, except that the lowest angle peak was observed at 4.4° (2 theta) corresponding to a d-spacing of 20.1A. The product had the following composition (mole ratio) and properties:

1.00TiO₂:0.41SiO₂:0.051Na₂O

Surface Area (m²/g) 275
Sorption (g/100g)

| | |
|---|---|
| H₂O | 12.9 |
| Cyclohexane | 9.3 |
| h-hexane | 7.1 |

### EXAMPLE 6

The procedure of Example 5 was repeated except that the n-octylamine swelling agent was replaced by 95.4g n-heptylamine. The resultant product had an x-ray diffraction pattern similar to those of Examples 1, 4 and 5 except that the lowest angle peak was observed at 4.0°(2 theta) or 22.1 angstrom d-spacing. The product had the following composition (mole ratios) and properties:

1.00TiO₂:0.55SiO₂:0.11Na₂O

Surface Area (m/²g) 241.0
Sorption (g/100g)

| | |
|---|---|
| H₂O | 10.9 |
| Cyclohexane | 4.5 |
| n-hexane | 5.1 |

### EXAMPLE 7

To a solution of 236.6g of 37.2% HCl in 525g of water were added 459.4g n-dodecylamine. The mixture was then transferred to a 2 liter polypropylene jar and heated at 100°C for 3 hours to obtain a homogeneous mixture. 75 grams of Na₂Ti₃O₇ were added, and the resulting mixture was heated at 100°C for 24 hours. The hot mixture was then diluted with 1 liter of hot water and allowed to filter overnight to dryness at room temperature. The product was re-slurried with 1 liter hot water, filtered, washed with 4 liters of hot water, and air-dried at room temperature for 24 hours.

30 grams of the dried product were stirred in 200g tetraethylorthosilicate at room temperature for 66 hours in a beaker covered with a watchglass. The product was filtered, dried at room temperature for 24 hours, and calcined in air for 5 hours at 538°C (1000°F) in a pre-heated oven.

The product had an x-ray diffraction pattern similar to those of Examples 1, 4, 5 and 6 except that the lowest angle peak was observed at 3.0° (2 theta) or 29.4 angstroms d-spacing. The product had the following composition (mole ratios) and properties:

1.00TiO₂:0.79SiO₂:0.047Na₂O

Surface Area (m²/g) 461
Sorption (g/100g)

| | |
|---|---|
| H₂O | 19.0 |
| Cyclohexane | 15.8 |
| n-hexane | 13.5 |

### EXAMPLE 8

Acid titanate, H₂Ti₃O₇, was prepared by hydrogen exchange of Na in Na₂Ti₃O₇ with 1 M HCl as described below: 780.7g of 37.4% HCl were diluted to 8 liters total volume with water in a 12 liter 4-necked round bottom flask equipped with a mechanical stirrer, reflux condenser, and thermometer. 500 grams of Na₂Ti₃O₇ were added, and the resulting mixture was heated with stirring at 75-80°C for 24 hours. The solution was then filtered and washed with 2 liters of hot water. The procedure was repeated three times. After the third exchange, the product was washed with hot water until chloride free. The product after drying in vacuo at 77°C had an x-ray diffraction pattern similar to that reported for H₂Ti₃O₇ by H. Izawa, S. Kikkaw, and M. Kolzumi, J. Phys. Chem., 86,5023 (1982).

The acid titanate were then swollen with n-octylamine as follows: 50g n-octylamine was dissolved in 150g of water in a 500 ml round-bottom flask equipped with a magnetic stirrer and reflux condenser. 10 grams of H₂Ti₃O₇ were added, and the resulting mixture was refluxed with stirring for 24 hours. The solid product was filtered, washed with 750 ml hot water, and air-dried at room temperature for 24 hours.

Eight grams of this dried product were stirred in 53g tetraethylorthosilicate for 67 hours at room temperature in a beaker loosely covered with clear plastic. The product was filtered, dried at room temperature for 24 hours, and calcined in air for 3 hours at 538°C (1000°F) in a pre-heated oven.

The final product had an x-ray diffraction pattern similar to those of Examples 1, 4, 5, 6 and 7, with the lowest angle peak observed at 5.0° (2 theta) or 17.9 angstroms d-spacing. The silicotitanate product of this Example had the following composition (mole ratios) and properties:

1.00TiO₂:0.19SiO₂:0.0067Na₂O

Surface Area (m₂/g) 276
Sorption (g/100g)

| | |
|---|---|
| H₂O | 11.5 |
| Cyclohexane | 8.2 |
| n-hexane | 8.2 |

### EXAMPLE 9

The acid titanate described in Example 8 was swollen with n-hexylamine as follows: 39.1g n-hexylamine were added to 150g water in a 500 ml round-bottom flask equipped with a magnetic stirrer and reflux condenser. 10 Grams of H₂Ti₃O₇ were added and the resulting mixture was refluxed with stirring for 24 hours. The solid product was filtered, washed with 750 ml hot water, and air dried at room temperature for 24 hours.

Eight grams of this dried product were stirred in 53g tetraethylorthosilicate for 72 hours at room temperature in a beaker loosely covered with clear plastic. The product was filtered, dried at room temperature for 24 hours, and calcined in air for 3 hours at 538°C (1000°F) in a pre-heated oven.

The final product had an X-ray diffraction pattern similar to those of Examples 1, 4, 5, 6, 7 and 8, with the lowest angle peak observed at 5.2° (2 theta) or 17.0 angstroms. The silicotitanate product of this Example had the following composition (mole ratios) and properties:

1.00TiO₂:0.22SiO₂:0.0062Na₂O

Surface Area (m₂/g) 201
Sorption (g/100g)

| | |
|---|---|
| H₂O | 8.8 |
| Cyclohexane | 5.7 |
| n-hexane | 5.5 |

### EXAMPLE 10

The acid titanate described in Example 8 was swollen with n-propylamine as follows: 22.9g n-propylamine were dissolved in 150g water in a 500 ml round-bottom flash equipped with a magnetic stirrer and reflux condenser. 10 Grams of H₂Ti₃O₇ were added and the resulting mixture was refluxed with stirring for 24 hours. The solid product was filtered, washed with 750 ml hot water, and air dried for 24 hours at room temperature.

Eight grams of this dried product were stirred in 53g of tetraethylorthosilicate for 72 hours at room temperature in a beaker loosely covered with clear plastic. The product was filtered, dried at room temperature for 24 hours, and calcined in air for 3 hours at 538°C (1000°F) in a pre-heated oven.

The final product had an X-ray diffraction pattern similar to those of Examples 1, 4, 5, 6, 7, 8 and 9, with the lowest angle peak observed at 8.7° (2 theta) or 10.2 angstroms d-spacing. The silicotitanate product of this Example had the following composition (mole ratios) and properties:

1.00TiO₂:0.21SiO₂:0.0068Na₂O

Surface Area (m²/g) 48
Sorption (g/100g)

| | |
|---|---|
| H₂O | 3.0 |
| Cyclohexane | 2.2 |
| n-hexane | 1.8 |

### EXAMPLE 11 (COMPARATIVE)

A 5 g natural magadiite sample from Trinity County, Ca. was dried for 3 hours at 110°C in air. The X-ray diffraction pattern of the dried sample showed a low angle peak at 5.6° (2 theta) indicating a basal spacing of 15.8A. The surface area was 28 m²/g by the nitrogen adsorption method. Other adsorption properties were 13% H₂O, 1.5% cyclohexane and 1.0% n-hexane. The alpha activity of the sample was 0.3. The sample was then exchanged twice with 10 ml/g of 0.1 N tetraethylammonium bromide at ambient temperature for 24 hours, filtered, water-washed and dried at 110°C for 3 hours. The surface area of the sample was 39 m²/g indicating very little interlaminar adsorption. The TEABr exchanged magadiite was thereafter calcined at 260°C for 2 hours. The x-ray pattern was similar to the dried unexchanged magadiite with basal spacing of 15.8A. The surface area of the sample was 36 m²/g.

5 g of natural magadiite was added to a solution of 82 ml of water and 18 ml of dilute Al₂(OH)Cl₅ solution with traces of silicate. The mixture was aged at room temperature for one hour with stirring. The solution was then heated to boiling for one hour and the solution pH adjusted to the 4.8-5.0 with 0.1 N NH₄OH. The sample was filtered, hot-water washed twice and dried. The surface area of the sample was 129 m²/g.

Twenty grams of the natural magadiite were sized to pass through a 25 mesh size screen and added to 100 ml of H₂O. The mixture was adjusted to a pH of about 2 and held there by addition of 0.1 N HCl within a 24 hour period. A total of 435 ml of 0.1N HCl was used. The sample was filtered, water-washed and dried. The x-ray pattern of the sample showed a peak at 7.8° evincing an 11.3A d-spacing indicating the structure was in a collapsed state. The calcined sample (3 hours at 538°C in air) had the same d-spacing. The adsorption properties were 27 m²/g surface area, 0.8% H₂O, 1.2% cyclohexane and 2.0% n-hexane.

### EXAMPLE 12 (COMPARATIVE)

Five grams of the acid treated and dried magadiite described in Example 11 were reacted with 10 g of dimethylsulfoxide and 6 g of octylamine for 24 hours at room temperature. The product was air-dried after decanting off excess liquid. The X-ray pattern exhibited a low angle (2 theta) peak at 2.7° indicating a basal or d-spacing of 32.7A. The air calcined (540°C) sample had a basal or d-spacing of 11.2 A, indicating the removal of the organic propping agent.

### EXAMPLE 13

One part of the uncalcined, octylamine-treated magadiite from Example 12 was treated with 8 parts of tetraethylorthosilicate for 24 hours at ambient temperature. The product was filtered, dried and calcined in air at 538°C for 2 hours. The X-ray pattern of the calcined product had a low angle (2 theta) peak at 4.6° relating to a basal or d-spacing of 19.2A. The adsorptive properties were: surface area 391 m²/g, H₂O 13.1%, cyclohexane 10.9% and n-hexane 10.6%. The alpha activity of the sample was 0.4.

### EXAMPLE 14

One part of the uncalcined, octylamine-treated magadiite from Example 12 was treated (to achieve a pH of about 2) by 8 parts of tetraethylorthosilicate and 0.4 parts of 0.1 N HCl with the same procedure as described in Example 14. After calcination at 538°C (1000°F), the lowest theta (2 theta) X-ray diffraction peak was 3.1° and basal or d-spacing 28.5 Angstroms. The surface area of the sample after calcination was 505 m²/g. Other adsorptive properties were: H₂O 18.6%, cyclohexane 15.5% and n-hexane 14.8%. The alpha activity of the sample was found to be 0.7. These properties indicate that a thermally stable and porous magadiite has been prepared.

### EXAMPLE 15

20 g Volclay-type bentonite were combined with 200g of water. The pH of the mixture was adjusted to and maintained at about 2 over 24 hours at room temperature by the intermittent addition of 0.1NHCl (145cc). The resulting acid-exchanged clay was washed with water, dried at room temperature, added to a mixture of 50g DMSO and 30g n-octylamine and reacted at room temperature for 24 hours whereupon it were filtered and dried. 100g of tetraethylorthosilicate was then added and the treatment at room temperature lasted for about 24 hours. The resulting product was calcined in air at 540°C for 3 hours. X-ray diffraction showed a broad low angle peak at about 3.5° corresponding to a basal spacing of about 25.2A. Surface area increased from 30 to about 324 m²/g. Water sorption increased from 1.0 to 10.7 wt.%, cyclohexane sorption increased from 0.5 to 10.9 wt.% and n-hexane increased from 0.8 to 8.9 wt%.

### EXAMPLES 16-19

A solution of 500 g Na₂Ti₃O₇ were refluxed with stirring in a solution of 427 g n-octylamine, 313 g 37.5% HCl and 7,000 g water for 22 hours in a 4-necked, round bottom flask equipped with a thermometer, reflux condenser, mechanical stirrer, and a stopper. The solution was decanted, reslurried with 2 liters hot water and decanted. An additional 2 liters of hot water were added and the resulting mixture was filtered and dried at room temperature for 24 hours. About 500 g of this product were slurried in 500 cc of absolute ethanol, filtered, and dried for one hour in air at room temperature. This product was reslurried in one liter water and heated with occasional stirring at 100^{o}C for 16 hours. This product was filtered and dried at room temperature for 24 hours.

400 g. of this product were mechanically stirred in 3000 g tetraethylorthosilicate in a 10 liter beaker covered with perforated aluminum foil for 72 hours at room temperature. This material was filtered and air-dried at room temperature for 24 hours. A 200 g portion of this product was calcined in nitrogen for 2 hours at 510^{o}C (950^{o}F) followed by air for one hour at 510^{o}C (950^{o}F). The silicotitanate product had a surface area of 273 m²/g and the following composition (wt. %):

| | |
|---|---|
| TiO₂ | 68.7 |
| SiO₂ | 24.8 |
| Na | 2.3 |

The material had an alpha-value of 2. After hydrothermal treatment (100% steam, 2 hours, 538^{o}C), the product had a surface area of 43 m²/g.

The product was then exchanged once with stirring with excess (9.8 moles metal salt/mole Na) 0.12 M solutions of each of the following metal salts at room temperature for 24 hours:

Ni(NO₃)₂.6H₂O; Cu(NO₃)₂.2-1/2 H₂O, Co(NO₃)₂.6H₂O,

Zn(NO₃)₂.6H₂O. After exchange, solutions were filtered, water washed, dried at 121^{o}C (250^{o}F) for 2 hours and calcined at 427^{o}C (800^{o}F) in air for one hour. Analyses were:

| Ex. | Surface Area (m²/g) | % Ni | % Cu | % Zn | % Co | % Na | % SiO₂ | % TiO₂ |
|---|---|---|---|---|---|---|---|---|
| 16 | 252 | 1.4 | - | - | - | 1.1 | 23.7 | 71.7 |
| 17 | 241 | - | 2.2 | - | - | 1.1 | 23.2 | 73.4 |
| 18 | 245 | - | - | 2.0 | - | 0.97 | 23.5 | 70.1 |
| 19 | 254 | - | - | - | 1.8 | 1.0 | 23.4 | 71.7 |

All exhibited high surface area after exchange of about half of the original sodium in the silicotitanate with divalent ions. Each sample was then steamed at 538^{o}C (1000^{o}F) for 2 hours (100% steam) with the following results:

| Example | | Surface Area (m²/g) After Steaming |
|---|---|---|
| 16 | | 90 |
| 17 | | 48 |
| 18 | 93 | |
| 19 | 86 | |

These results indicate improved hydrothermal stability as a result of Ni, Zn, or Co exchange.

### Example 20

A mixture of 500 g Na₂Ti₃O7, 427 g n-octylamine, 309.7, 37.8% HCl and 7000 g water was refluxed for 2.2 hours as in Example 18. The solution was decanted and filtered and dried on the filter at room temperature overnight. This product was then treated twice with absolute ethanol and water as follows: the solid product was reslurried in 2 liters ethanol, filtered, and air-dried 6 hours at room temperature. This material was then slurried in 1.5 liters water, heated at 100^{o}C in a 2 liter polypropylene jar for 17 hours, filtered, and dried at room temperature for 24 hours.

450 g of the dried product were mechanically stirred in 3000 g of tetraethylorthosilicate in a 10 liter beaker covered with perforated aluminum foil for 68 hours at room temperature and then filtered and dried in air at room temperature for about 4 days. This material was calcined in nitrogen at 510^{o}C (950^{o}F) for 2 hours and then in air for one hour at 510^{o}C (950^{o}F). The silicotitanate product had a surface area of 405 m²/g and the following composition (wt. %):

| | |
|---|---|
| TiO₂ | 51.7 |
| SiO₂ | 39.9 |
| Na | 1.8 |

This material had an alpha-value of 3. After hydrothermal treatment (100% steam, 2 hours, 538^{o}C), the product had a surface area of 62 m²/g.

Three grams of the resulting silicotitanate were added to 125 ml of 0.1 N CsCl solution. The mixture was stirred in a polyproylene bottle at ambient temperature for 7 days. The solid was separated by filtration and was then water washed to chloride free and dried in vacuum. The exchanged sample was analyzed and found to contain 0.82% Na and 4.35% Cs by wt. About half of the original sodium was exchanged by the cesium ions.

### Examples 21 and 22

Further samples of the silicotitanate produced in Example 20 were exchanged four times with stirring with excess (4.6 moles metal salt/mole Na) 0.10 M solutions of the following salts: Al(NO₃)₃.9H₂O and La(NO₃)₃.6H₂O. Samples were filtered and water-washed after each exchange; after the final exchange, both samples were calcined in air at 510^{o}C (850^{o}F). Surface areas and chemical analyses were:

| Ex. | Surface Area (m²/g) | % Al | % La | % Na | % SiO₂ | % TiO₂ |
|---|---|---|---|---|---|---|
| 21 | 355 | 0.49 | - | 0.77 | 42.1 | 55.9 |
| 22 | 374 | - | 2.05 | 0.70 | 42.5 | 56.2 |

Slightly more than half of the original sodium was exchanged by the trivalent ions, and high surface area was retained. The product of Example 21 had an alpha-value of 6, indicating considerable activation as a result of exchange of Na by Al. Each example was steamed (100% steam, 2 hours 1000^{o}F) with the following results:

| Example | | Surface Area (m²/g) After Steaming |
|---|---|---|
| 21 | | 262 |
| 22 | 198 | |

Thus, trivalent ion exchange dramatically improved hydrothermal stability.

### Example 23

This example demonstrates exchange of sodium in a silicotitanate with a tetravalent ion (ceric ion). The silicotitanate product in Example 20 was exchanged once with stirring with excess (9.9 moles metal salt per Na) 0.1 M Ce (SO₄)₄ .2H₂SO₄ at room temperature for 24 hours. The solution was filtered, water-washed, dried at 121^{o}C (250^{o}F), and calcined at 427^{o}C (800^{o}F) in air for one hour. The product had a surface area of 378 m²/g and the following composition (wt. %):

| | |
|---|---|
| TiO₂ | 43.0 |
| SiO₂ | 37.6 |
| Na | 0.74 |
| Ce | 1.9 |

### Example 24

a) A gel was produced by mixing 400 g Cabosil silica in 54.4 g 98% NaOH and 1.4 kg water. The gel was crystallized in a 2 liter polypropylene jar at 100^{o}C for 23 days to produce synthetic magadiite, which was then filtered, washed with hot water and dried at (250^{o}F) overnight. The dried product had the following composition (wt%):

| | |
|---|---|
| SiO₂ | 83.3 |
| Na₂O | 6.9 |
| Al₂O₃ | 0.01 |

100 g of the dried product was added to 600 ml of distilled water, titrated with 0.1 N HCl to a pH of 2, and held at pH of 2 for 24 hours. The product, after being filtered, washed with 8 liters of distilled water, and air dried on the filter, had 95 ppm Na.
The resultant product (80 g) was treated for 24 hours with a solution of 80 g of octylamine in 160 g of DMSO, filtered, air dried and then held for subsequent treatments.
b) A solution of tetraethylorthosilicate (TEOS) and aluminum isopropoxide (AIP) was prepared as follows:
80 g of aluminum isopropoxide (30-35%) in isobutanol (Alfa) were placed in a 250 ml polypropylene bottle and heated in a steam chest at 100^{o}C for 16 hours. 51.0 g TEOS (Baker, practical grade) were added and this solution was stirred for 3 days at room temperature.
20 g of the octylamine propped product of (a) above were reacted with the TEOS/AIP solution for 3 days in a polypropylene bottle which was tightly sealed. The slurry was filtered, air dried, and calcined for 2 hours at 510^{o}C (950^{o}F) in air. The final product had an alpha = 5 and the following composition (wt. %):

| | |
|---|---|
| SiO₂ | 72.90 |
| Al₂O₃ | 16.8 |

### Examples 25-26

Further 20 g samples of the propped product of Example 24(a) were reacted respectively with 100 g samples of titanium tetraisopropoxide (Example 25) and tetraethylorthosilicate (Example 26). Each reaction was conducted at room temperature for 3 days in a sealed polypropylene bottle, whereafter the resultant slurry was filtered, air-dried and calcined for 2 hours at 538^{o}C (1000^{o}F) in air. The products bad the following properties:

| Example | Alpha | Composition (wt %) | | |
|---|---|---|---|---|
| | | SiO₂ | Al₂O₃ | Ti |
| 25 | 3 | 53.7 | 0.015 | 27 |
| 26 | 1 | 94 | 0.0025 | - |

### EXAMPLE 27

a) 110 g of the acid form of synthetic magadiite prepared in a manner analogous to Example 24 were treated with a solution of 150 g of octylamine in 300 g of distilled water for 24 hours at room temperature. The slurry was filtered to a wetcake, reslurried (285 g of wetcake in 5.7 liters of distilled water), left for approximately 1 hour at room temperature, and refiltered. The product was composed of 238 g of paste-like material (41.54% solids).
b) 294.2 g of aluminum isopropoxide (30-35%) in isobutanol were placed in a polypropylene bottle in a steam chest (100^{o}C) overnight. 171.6 g of solution was recovered after overnight heating. 220 g of tetraethylorthosilicate were added to the aluminum isopropoxide solution and the mixture was magnetically stirred for 9 days at room temperature.
c) The product (b) was added to the product (a) then an additional 400 g of fresh tetraethylorthosilicate were added. This mixture was reacted for 65 hours at room temperature in a sealed polypropylene bottle with magnetic stirring. The slurry was filtered with difficulty, air dried, dried overnight at 110^{o}C and then calcined at 538^{o}C for 1 hour in flowing nitrogen followed by 2 hours in flowing air. The final product had an alpha = 10 and the following composition (wt %):

| | |
|---|---|
| SiO₂ | 83.1 |
| Al₂O₃ | 8.7 |

The surface area and sorption properties of the calcined magadiites obtained in Examples 24-27 are summarized in the following table:

| Example | Surface Area m²/g | Sorption Capacity | | |
|---|---|---|---|---|
| | | H₂O (12 Torr) | Cy-C₆ (40 Torr) | n-C₆ (40 Torr) |
| 24 | 289 | 14.2 | 8.2 | 4.7 |
| 25 | 158 | 9.2 | 4.4 | 3.3 |
| 26 | 307 | 7.2 | 6.0 | 4.3 |
| 27 | 450 | 16.3 | 12.3 | 10.9 |

## Claims

1. A method for preparing a layered product having adjacent layers separated by pillars of a polymeric oxide of an element selected from Groups IB, IIB, IIIA, IIIB, IVA, IVB, VA, VB, VIA, VIIA, and VIIIA of the Periodic Table, which method comprises starting with a layered oxide material of an element ranging in atomic number from 13 to 15, 21 to 33, 39 to 51, 57 to 83 and greater than 90, said layered oxide material having interlayer cations associated therewith; physically separating the layers of the oxide material by introducing an organic cationic species between the layers at anionic sites; thereafter introducing between the separated layers of said layered oxide at least one neutral compound capable of conversion to a polymeric oxide; and converting said compound to the said polymeric oxide to produce pillars of the polymeric oxide separating adjacent layers of the layered oxide material.

2. The method of claim 1 wherein said organic cationic species is an alkylammonium cation having at least 3 carbon atoms.

3. The method of claim 2 wherein said alkylammonium cation is derived from an n-alkyl primary monoamine.

4. The method of any preceding claim wherein said neutral compound capable of conversion is hydrolyzable into said polymeric oxide and said conversion step comprises hydrolysis of said compound.

5. The method of any preceding claim wherein said at least one compound capable of conversion is an organic compound of silicon, germanium, tin, lead, zirconium, titanium and/or aluminum.

6. The method of any preceding claim wherein said at least one compound is a tetraalkylsilicate.

7. The method of claim 6 wherein said at least one compound is a tetraalkylsilicate and an aluminum alkoxide.

8. The method of any preceding claim wherein the layered oxide is an oxide of silicon or titanium.

9. The method of any preceding claim wherein the layered oxide is non-swellable.

10. The method of any one of claims 1 to 8 wherein the layered oxide is a clay.

11. A layered product comprising a non-swellable layered oxide of an element ranging in atomic numbers from 13 to 15, 21 to 33, 39 to 51, 57 to 83 and greater than 90 inclusive, and pillars containing at least one polymeric oxide separating the oxide layers.

12. The layered product of claim 11 having a d-spacing greater than 20A.

13. The composition of claim 11 or claim 12 wherein said layered oxide is an oxide of a metal selected from silicon, titanium, zirconium, and hafnium.

14. The layered product of any one of claims 11 to 13 wherein the layered oxide is a layered silicate.

15. The layered product of claim 14 wherein the pillars comprise aluminum oxide.

16. A layered titanate composition comprising pillars of polymeric silica between the titanium oxide layers and having the x-ray diffraction pattern set out below.
| 2Θ min - 2Θmax | 100 I/Iₒ (Relative Intensity) Range |
|---|---|
| ≦ 8.7 | vs to W |
| 11.1 - 14.3 | S to W |
| 11.8 - 15.2 | M to W |
| 24.5 - 25.0 | VS to W |
| 25.0 - 25.4 | M to W |
| 28.5 - 30.2 | vs to W |
| 29.8 - 30.6 | S to W |
| 33.0 - 33.5 | S to W |
| 43.2 - 43.5 | M to W |
| 44.2 - 44.7 | M to W |
| 48.5 - 48.9 | vs to M |
| 52.7 - 52.9 | W |

## Patentansprüche

1. Verfahren zur Herstellung eines Schichtproduktes mit benachbarten Schichten, die durch Stützen eines polymeren Oxids eines Elementes getrennt sind, das aus den Gruppen IB, IIB, IIIA, IIIB, IVA, IVB, VA, VB, VIA, VIIA und VIIIA des Periodensystems ausgewählt ist, wobei dieses Verfahren das Beginnen mit einem Schichtoxidmaterial eines Elementes im Bereich der Atomzahl von 13 bis 15, 21 bis 33, 39 bis 51, 57 bis 83 und mehr als 90, wobei dieses Schichtoxidmaterial damit verbundene Zwischenschichtkationen aufweist; die physikalische Trennung der Schichten des Oxidmaterials durch Einführung organischer kationischer Arten zwischen die schichten an den anionischen Plätzen; die anschließende Einführung von zumindest einer neutralen Verbindung zwischen die getrennten Schichten des Schichtoxids, die sich in ein polymeres Oxid umwandeln kann; und die Umwandlung der Verbindung in das polymere Oxid umfaßt, um die Stützen des polymeren Oxids zu bilden, die die benachbarten Schichten des Schichtoxidmaterials trennen.

2. Verfahren nach Anspruch 1, worin die organischen kationischen Arten ein Alkylamoniumkation mit mindestens drei Kohlenstoffatomen sind.

3. Verfahren nach Anspruch 2, worin das Alkylamoniumkation von einem primären n-Alkylmonoamin abgeleitet ist.

4. Verfahren nach einem der vorstehenden Ansprüche, worin die umwandelbare neutrale Verbindung in das polymere Oxid hydrolysierbar ist und der Umwandlungsschritt die Hydrolyse der Verbindung umfaßt.

5. Verfahren nach einem der vorstehenden Ansprüche, worin die zumindest eine umwandelbare Verbindung eine organische Verbindung von Silicium, Germanium, Zinn, Blei, Zirconium, Titan und/oder Aluminium ist.

6. Verfahren nach einem der vorstehenden Ansprüche, worin die zumindest eine Verbindung Tetraalkylsilicat ist.

7. Verfahren nach Anspruch 6, worin die zumindest eine Verbindung Tetraalkylsilicat und ein Aluminiumalkoxid ist.

8. Verfahren nach einem der vorstehenden Ansprüche, worin das Schichtoxid ein Oxid von Silicium oder Titan ist.

9. Verfahren nach einem der vorstehenden Ansprüche, worin das Schichtoxid nicht quellbar ist.

10. Verfahren nach einem der Ansprüche 1 bis 8, worin das Schichtoxid Ton ist.

11. Schichtprodukt, das ein nicht quellbares Schichtoxid eines Elementes im Bereich der Atomzahl von 13 bis 15, 21 bis 33, 39 bis 51, 57 bis 83 und mehr als einschließlich 90 und Stützen umfaßt, die mindest ein polymeres Oxid enthalten und die Oxidschichten trennen.

12. Schichtprodukt nach Anspruch 11 mit einem d-Abstand von mehr als 20 Å.

13. Zusammensetzung nach Anspruch 11 oder 12, worin das Schichtoxid ein Oxid eines Metalls ist, das aus Silicium, Titan, Zirconium und Hafnium ausgewählt ist.

14. Schichtprodukt nach einem der Ansprüche 11 bis 13, worin das Schichtoxid ein Schichtsilicat ist.

15. Schichtprodukt nach Anspruch 14, worin die Stützen Aluminiumoxid umfassen.

16. Schichttitanat-Zusammensetzung, die Stützen eines polymeren Siliciumdioxids zwischen den Titanoxidschichten umfaßt und das nachfolgende Röntgenbeugungsdiagramm aufweist.
| 2Θ min. - 2Θ max. | 100 I/I₀-Bereich (Relative Intensität) |
|---|---|
| ≦ 8,7 | VS bis W |
| 11,1 - 14,3 | S bis W |
| 11,8 - 15,2 | M bis W |
| 24,5 - 25,0 | VS bis W |
| 25,0 - 25,4 | M bis W |
| 28,5 - 30,2 | VS bis W |
| 29,8 - 30,6 | S bis W |
| 33,0 - 33,5 | S bis W |
| 43,2 - 43,5 | M bis W |
| 44,2 - 44,7 | M bis W |
| 48,5 - 48,9 | VS bis M |
| 52,7 - 52,9 | W |

## Revendications

1. Un procédé pour la préparation d'un produit stratifié comportant des couches adjacentes séparées par des piliers d'oxyde polymère d'un élément choisi dans les groupes IB, IIB, IIIA, IIIB, IVA, IVB, VA, VB, VIA, VIIA et VIIIA de la table périodique, ce procédé consistant:
- à partir d'un matériau formé d'oxyde stratifié d'un élément ayant un nombre atomique compris entre 13 à 15, 21 à 33, 39 à 51, 57 à 83 et supérieur à 90, ledit matériau formé d'oxyde stratifié présentant des cations intermédiaires qui leur sont associés;
- à séparer physiquement les couches du matériau formé d'oxyde en introduisant un composé cationique organique entre les couches sur les sites anioniques;
- à introduire entre les couches séparées desdits oxydes stratifiés au moins un composé neutre susceptible d'être converti en oxyde polymère; et
- à convertit ledit composé neutre en ledit oxyde polymère pour produire des piliers d'oxyde polymère séparant les couches adjacentes du matériau formé d'oxyde stratifiés.

2. La méthode selon la revendication 1, dans laquelle le composé cationique organique est un cation alkylammonium ayant au moins 3 atomes de carbone.

3. La méthode selon la revendication 2, dans laquelle ledit cation alkylammonium est dérivé d'une monoamine primaire n-alkylée.

4. La méthode selon l'une quelconque des revendications précédentes, dans laquelle ledit composé neutre capable de conversion est hydrolysable en ledit oxyde polymère et ladite étape de conversion comprend l'hydrolyse dudit composé neutre.

5. La méthode selon l'une quelconque des revendications précédentes, dans laquelle un desdits composés au moins capable de conversion est un composé organique de silicium, germanium, étain, plomb, zirconium, titane et/ou aluminium.

6. La méthode selon l'une quelconque des revendications précédentes, dans laquelle l'un desdits composés au moins est un tétraalkylsilicate.

7. La méthode selon la revendication 6, dans laquelle desdits composés au moins est un tétraalkylsilicate et un aluminium alcoxyde .

8. La méthode selon l'une quelconque des revendications précédentes, dans laquelle l'oxyde stratifié est un oxyde de silicium ou de titane.

9. La méthode selon l'une quelconque des revendications précédentes, dans laquelle l'oxyde stratifié est non gonflable.

10. La méthode selon l'une quelconque des revendications 1 à 8, dans laquelle l'oxyde stratifié est une argile.

11. Un produit stratifié comprenant un oxyde stratifié non gonflable d'un élément compris dans les nombres atomiques de 13 à 15, 21 à 33, 39 à 51, 57 à 83 et supérieurs à 90 bornes incluses, et des piliers contenant au moins un oxyde polymère séparant les couches d'oxyde.

12. Le produit stratifié de la revendication 11, ayant un espacement d supérieur à 20 Å.

13. La composition selon la revendication 11 ou 12, dans laquelle ledit oxyde stratifié est un oxyde d'un métal choisi parmi le silicium, le titane, le zirconium et l'hafnium.

14. Le produit stratifié selon l'une des revendications 11 à 13, dans lequel l'oxyde stratifié est un silicate stratifié.

15. Le produit stratifié de la revendication 14, dans lequel les piliers comprennent un oxyde d'aluminium.

16. Une composition de titanate stratifié comprenant des piliers de silice polymère entre les couches d'oxyde de titane et présentant le spectre de diffraction aux rayons X représenté ci-dessous:
| 20 min - 20 max | 100 I/Iₒ gamme (intensité relative) |
|---|---|
| ≦ 8,7 | VS à W |
| 11,1 à 14,3 | S à W |
| 11,8 à 15,2 | M à W |
| 24,5 à 25,0 | VS à W |
| 25,0 à 25,4 | M à W |
| 28,5 à 30,2 | VS à W |
| 29,8 à 30,6 | S à W |
| 33,0 à 33,5 | S à W |
| 43,2 à 43,5 | M à W |
| 44,2 à 44,7 | M à W |
| 48,5 à 48,9 | VS à M |
| 52,7 à 52,9 | W |
